# EUROPEAN PATENT APPLICATION

(11) **EP 3 712 128 A1**
(43) Date of publication of application: **23.09.2020**
(21) Application number: 19382189.9
(22) Date of filing: 18.03.2019
(51) Int. Cl.: C07D 207/34, C07C 303/40, C07D 209/88

(54) **PHOTOCHEMICAL INTERMOLECULAR AMINATION METHOD**

(71) Applicant: Fundació Institut Català d'Investigació Química (ICIQ), 43007 Tarragona (ES); Institució Catalana de Recerca i Estudis Avançats (ICREA), 08010 Barcelona (ES)
(72) Inventor: MUÑIZ KLEIN, Kilian, 43007 Tarragona (ES); BOSNIDOU, Alexandra, 43002 Tarragona (ES)

(57) **Abstract**

**Problem:** The present invention solves the problem of providing an alternative metal-free method for intermolecular amination of C-H bonds applicable to a broad range of substrates, wherein the substrate is essentially in stoichiometric amounts with respect to the amination reagent.

**Solution:** The present invention relates to a photochemical method for the intermolecular coupling of an amination agent with a compound comprising a fragment of formula -Cₐ-H, said method converting the fragment of formula - Cₐ-H in a fragment of formula -Cₐ-NSO₂R₁, and comprising the step of contacting under light irradiation and in an aprotic solvent an amination agent with a compound comprising a fragment of formula -Cₐ-H in the presence of a compound of formula (I)

Arl(O₂CR₃)₂ (I)

and a catalytically effective amount of iodine wherein the amination agent is selected from the compounds of formula R₁SO₂NHR₂ and the compounds of formula R₁SO₂N=IR₂'.

## Description

The present invention belongs to the field of chemistry, more particularly to the field of organic synthesis and photochemistry and to the field of synthetic preparation of aminated compounds. It relates more particularly to a metal-free, photochemical method for the intermolecular insertion of a sulfonamide moiety into a C-H bond, particularly into a benzylic C-H bond, producing aminated products.

### BACKGROUND ART

A large number of pharmaceutical ingredients or biologically active natural products comprise a nitrogen-containing functional group in their molecular structure. For this reason, methods for the introduction of such functional groups within molecular structures are of great interest to synthetic chemists. The amine group is not only useful to bring further complexity in a structure through amine reactivity, but also presents interesting electronic properties responsible for interactions with binding pockets of biomolecules (such as proteins, enzymes, etc). In particular, benzylic amines, i.e. amine groups attached to a carbon atom that is adjacent to an aromatic or heteroaromatic group in a compound, represent a common scaffold in certain active ingredients (such as memantine, maraviroc, MK-801, sertraline, rasagiline, akuammiline alkaloids, repaglinide, cinacalcet, prasugrel, flunarizine, solifenacin, aryl glycine, and hexafluronium). The synthetic chemistry community thus has a strong interest in developing methods for the preparation of amines or of benzylic amines. The development of synthetic methods for the insertion of an amine group into a C-H bond, wherein the carbon atom is a saturated carbon atom is of particular interest.

Several methods have been reported allowing for the preparation of amines by insertion of a nitrogenated group into a C-H bond. In particular, the insertion of a nitrogenated functional group as amine precursor into an unactivated aliphatic C-H bond is a synthetic challenge. To this end, nitrene insertion into a C-H bond has been explored to some extent. Such methods typically involve the use of reagents containing an azide group, which hampers the implementation of such method in the large scale, due to the well-known explosive character of azide compounds. Alternatively, hypervalent iodine compounds such as [N-(p-toluenesulfonyl)imino]-phenyliodinane have been developed for this chemistry. Methods for nitrene insertion based on such hypervalent iodine reagents commonly require the use of catalysts based on transition metals to mediate the reaction and induce site-selectivity. The use of metal-based processes is undesirable in certain fields of application as residual traces of metals in the final product need to be avoided in order to comply with product-related regulations.

Metal-free methods for the amination of aliphatic C-H bonds have thus been developed in the state of the art. These methods however suffer from a narrow scope of applicability as relates to the substrate of the reaction. For instance, Bergès and co-workers have reported a method for the amination of indole-based substrates involving the use of phthalimide as amine source together with a hypervalent iodine compound and a soluble bromide salt as catalyst (in the form of a quaternary ammonium). Such method goes through the formation of a bromine(I) compound linked to two phthalimide units, suitable to transfer a bromide group at position 3 of the indole scaffold, which then reacts with a phthalimide anion to form the nitrogenated product. According to the suggested mechanism of the reaction, this method requires the presence of the indole scaffold on the substrate of reaction.

In a different approach, Duhamel and co-workers have reported a method for the preparation of amines (such as pyrrolidines) by cyclization of substrates containing nitrogenated alkyl fragments. The described method, being limited to intramolecular reactions, suffers from a narrow substrate scope regarding the reactive C-H bonds. The cyclization method comprises the step of contacting the substrate of reaction with a catalytic amount of iodine and an oxidant (being either a peracid or iodine(III) species) under light irradiation.

Souto and co-workers reported in 2012 a method for the intermolecular amination of allylic substrates. The method comprises the step of contacting the allylic substrate with bis-sulfonamide in the presence of a hypervalent iodine reagent bearing a bis-sulfonamide group. This method is limited to allylic substrates and allows for the preparation of bis-sulfonamide compounds. The preparation of the free amine from the bis-sulfonamide by hydrolysis of the sulfonamide groups generally requires harsh conditions of reaction, which also limits the scope of substrates for this reaction.

Lamar and co-workers reported in 2010 a method for the amination of C-H bonds, wherein said method comprises contacting a substrate comprising a C-H bond reactive to amination conditions with a compound of formula PhINQ, being Q an arylsulfonyl group (such as tosyl or nosyl) in the presence of a catalytic amount of iodine. According to the authors, the method produces good yields of aminated product (superior to 50%) when Q is a nosyl group or when either the substrate comprising the C-H bond or the amination agent is used in excess amounts. Best reported conditions include the use of PhlNNs as amination agent together with a large excess (5 equivalents) of the substrate comprising the C-H bond. In such conditions, the maximum achievable yield of the amination product with respect to the substrate comprising the C-H bond is 20%.

Fan and his collaborators reported in 2009 a method for the intermolecular amination of benzylic substrates. The reported method comprises the step of heating a benzylic substrate with a sulfonamide in the presence of iodine and of phenyl iodine(III) diacetate as an oxidant. According to the authors, reasonable yields for the method are obtained when the substrate is used as a solvent and in large excess (at least 10 times the amount of sulfonamide). In such conditions, the maximum achievable yield of the amination product with respect to the substrate comprising the C-H bond is 10%. These limitations on the substrate narrow the scope of applicability of the method to substrates which are liquid in the conditions of the reaction. In addition, it is reported that the reaction takes place only when the mixture is heated and that irradiation with light allows increasing the kinetics of the reaction.

From what is known in the art, it derives that there is still a need for providing alternative metal-free methods for intermolecular amination of C-H bonds applicable to a broad range of substrates, wherein the substrate is essentially in equimolar amounts with respect to the amination reagent.

### SUMMARY OF THE INVENTION

The inventors have developed a method for the intermolecular amination of substrates, said method comprising the step of contacting the substrate with a sulfonamide compound under light irradiation in the presence of a catalytic amount of iodine and in the presence of a (dicarboxylato)iodobenzene compound wherein the carboxylate group is an aryl carboxylate compound or a haloalkylcarboxylate. The inventors found that the combination of visible light irradiation with the use of certain (dicarboxylato)iodobenzene compounds as oxidants unexpectedly allows to use the reaction substrate in less amount, which advantageously enables the use of a solvent and provides a method that is broader in terms of substrate scope than the methods described in the state of the art, in the extent that substrates that are solid in the conditions of the reaction may be employed. The method of the invention advantageously takes place under metal-free and mild conditions, at room temperature and using a solvent, which makes it applicable to a broader range of substrates than the methods described in the state of the art.

Thus, in a first aspect, the invention relates to a photochemical method for the intermolecular coupling of an amination agent with a compound comprising a fragment of formula -Cₐ-H, said method converting the fragment of formula -Cₐ-H in a fragment of formula -Cₐ-NSO₂R₁, and comprising the step of contacting under light irradiation and in an aprotic solvent an amination agent with a compound comprising a fragment of formula -Cₐ-H in the presence of a compound of formula (I)

Arl(O₂CR₃)₂ (I)

and a catalytically effective amount of iodine, wherein:
the amination agent is selected from the compounds of formula R₁SO₂NHR₂ and the compounds of formula R₁SO₂N=IR₂' wherein:
   R₁ is a radical selected from the group consisting of (C₁-C₆)alkyl, (C₁-C₆)haloalkyl, and (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy;
   R₂ is selected from hydrogen and a (C₁-C₆)alkyl group;
   R₂' a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy;
Cₐ is a saturated carbon atom;
Ar is a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy;
R₃ is a radical selected from the group consisting of (C₁-C₆)haloalkyl and a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxycarbonyl, and (C₁-C₆)alkylcarbonyloxy;
and the amount of the compound of formula (I) is at least twice the amount of the compound comprising the fragment of formula -Cₐ-H.

The method of the invention is particularly useful for amination reaction in benzylic positions, i.e. at carbon atoms, which are adjacent to aromatic or heteroaromatic groups in the reaction substrate. The benzylic amine scaffold is common to numerous structures of pharmaceutical ingredients or other biologically active natural products.

The invention therefore relates in a second aspect to a method for the preparation of a biologically active compound or a salt thereof comprising the step of carrying out the photochemical method according to the first aspect of the invention.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges given, such as temperatures, times, molar ratio and the like, should be considered approximate (i.e. with a 5% margin of variation around indicated point), unless specifically stated.

In the context of the invention, the term "amination" refers to the introduction of a nitrogenated functional group, such as a sulfonamido group, an amido group, an amino group, a nitro group, into a carbon atom of a reaction substrate.

In the context of the invention, the term "alkyl" refers to a saturated, linear or branched, hydrocarbon chain having the number of carbon atoms indicated in the description or in the claims. For instance, the term (C₁-C₆)alkyl refers to a saturated, linear or branched hydrocarbon chain having form one to six carbon atoms, such as methyl, ethyl, propyl, iso-propyl, butyl, tert-butyl, iso-butyl, pentyl and hexyl.

In the context of the invention, the term "haloalkyl" refers to a saturated, linear or branched, hydrocarbon chain having the number of carbon atoms indicated in the description or in the claims and wherein at least one of the hydrogen groups is replaced by a halo group, such as fluoro, chloro, bromo and iodo. It is preferred in the context of the invention that the term "haloalkyl" refers to a "perfluoroalkyl", that is an alkyl group as defined above wherein all the hydrogen atoms are replaced by fluorine atoms, such as, for instance, in a trifluoromethyl group.

In the context of the invention, the term "alkyloxy" refers to a saturated, linear or branched, hydrocarbon chain having the number of carbon atoms indicated in the description or in the claims and attached to the remainder of the molecule through an oxy (-O-) group.

In the context of the invention, the term "alkyloxycarbonyl" refers to a saturated, linear or branched, hydrocarbon chain having the number of carbon atoms indicated in the description or in the claims and attached to the remainder of the molecule through an oxycarbonyl (-O(CO)-) group.

In the context of the invention, the term "alkylcarbonyloxy" refers to a saturated, linear or branched, hydrocarbon chain having the number of carbon atoms indicated in the description or in the claims and attached to the remainder of the molecule through a carbonyloxy (-(CO)O-) group.

In the context of the invention, the term "aryl" refers to an aromatic hydrocarbon ring system comprising the number of carbon atoms indicated in the description and claims and comprising 5 to 6-membered rings. Examples of aryl groups include, but are not limited to: phenyl, naphthyl, indenyl, anthracenyl and phenanthrenyl.

In the context of the invention, the term "heteroaryl" refers to an aromatic ring system comprising the number of atoms indicated in the description and claims and comprising 5 to 6-membered rings, the members of said rings being selected from the group consisting of C, N, O, and S, said members being substituted, where chemically possible, with a radical selected from the group consisting of hydrogen, alkyl, alkyloxy, haloalkyl, alkyloxycarbonyl, alkylcarbonyloxy, cyano and nitro.

In the context of the invention, the term "saturated carbon atom" refers to a carbon atom being connected to four atoms through single covalent bonds. In a similar manner, the term "unsaturated carbon atom" refers to a carbon atom that is connected to two or three atoms through a combination of single, double or triple bonds, provided that at least one or two of the connections is a double or triple bond.

In the context of the invention, the term "catalytically effective amount" refers to the amount of a catalyst system or compound that is sufficient to trigger the catalytic transformation at rate higher than the rate of the uncatalyzed transformation. Generally, this amount is sub-stoichiometric, which means that the catalysts system or compound is used in minor amounts than the substrate of the reaction.

In the context of the invention, the term "intermolecular", when related to a method, refers to the fact that the chemical reaction of the method takes place between two separate molecules, said molecules not being linked one with the other. In addition, the term "intermolecular coupling" refers to a chemical reaction wherein a multiplicity, preferably two, of initially non-connected molecules react together to form one chemical entity in such a way that a covalent bond is created between the initially non-connected molecules.

In the context of the invention, the term "benzylic carbon atom" refers to a carbon atom that is adjacent to an aryl or heteroaryl group as defined above.

In the context of the invention, the term "kinetic resolution method" refers to a method, in which an equal mixture of enantiomers of a compound with a ratio of 50:50 is converted into an enantiomerically enriched compound, wherein an "enantiomerically enriched compound" refers to a composition of a chiral compound containing one of its enantiomers in a ratio other than 50:50.

The term "primary carbon atom" refers to a saturated carbon atom that is connected to four atoms, three of said atoms being hydrogen. The term "secondary carbon atom" refers to a saturated carbon atom that is connected to four atoms, two of said atoms being hydrogen. The term "tertiary carbon atom" refers to a saturated carbon atom that is connected to four atoms, one of said atoms being hydrogen. The term "quaternary carbon atom" refers to a saturated carbon atom that is connected to four atoms, none of said atoms being hydrogen.

According to the first aspect of the invention, the invention relates to a photochemical intermolecular amination method as defined above, producing a compound comprising a fragment of formula -Cₐ-NSO₂R₁ as defined above. The method of the invention comprises the step of contacting under light irradiation and in an aprotic solvent an amination agent selected from the compounds of formula R₁SO₂NHR₂ and the compounds of formula R₁SO₂N=IR₂'with a compound comprising a fragment of formula -Cₐ-H in the presence of a compound of formula (I)

Arl(O₂CR₃)₂ (I)

and a catalytically effective amount of iodine, wherein R₁, R₂, R₂', R₃ and Ar are as defined above. Without being bound to theory, it is believed that the method of the invention may be divided into three main steps:
(i) iodination step wherein the fragment of formula -Cₐ-H is converted under light irradiation in a fragment of formula -Cₐ-I;
(ii) oxidation step wherein the product of step (i) is oxidized by the compound of formula (I), producing a compound comprising a fragment of formula -Cₐ-I(O₂CR₃)₂, and
(iii) amination step wherein the product of step (ii) is converted into a compound comprising a fragment of formula -Cₐ-NSO₂R₁ by reaction with the amination agent.

It is believed that step (i) of the method of the invention may involve the formation of the compound comprising a fragment of formula -Cₐ^{•}. Thus, when the compound comprising the fragment of formula -Cₐ-H comprises more fragments of formula -C-H, the formation of the most stable free radical shall be favored and Cₐ is essentially the carbon atom of the compound comprising a fragment of formula -Cₐ-H that is most suited for the stabilization of a free radical. The person skilled in the art shall be able to determine which carbon atom of a compound is most suited for the stabilization of a free radical using common general knowledge, such as the one disclosed in Jonathan Clayden, Organic Chemistry 2nd edition, chapter 39 (ISBN: 978-0199270293, Oxford University Press).

In preferred embodiments of the first aspect of the invention, Cₐ is essentially the carbon atom of the compound comprising a fragment of formula -Cₐ-H that is most suited for the stabilization of a free radical.

As it is known in the art, certain groups are suitable for the stabilization of free radicals, this is particularly the case when the free radical may be delocalized by electronic conjugation with adjacent unsaturated systems, such as double bonds, aryl or heteroaryl groups. In more preferred embodiments of the first aspect of the invention, optionally in combination with one or more features of the various embodiments described below, Cₐ is a benzylic carbon atom.

In other preferred embodiments of the first aspect of the invention, optionally in combination with one or more features of the various embodiments described below, Cₐ is a carbon atom adjacent to the nitrogen atom of a functional group selected from the group consisting of amido, carbamate, sulfonamide and phosphoramide. In more preferred embodiments, optionally in combination with one or more features of the various embodiments described below, Cₐ is a carbon atom adjacent to the nitrogen atom of a carbamate functional group.

In certain preferred embodiments of the first aspect of the invention, optionally in combination with one or more features of the various embodiments described above and below, Cₐ is a secondary carbon atom. Cₐ is preferably a secondary carbon atom when the compound comprising the fragment of formula Cₐ-H comprises more than one fragment of formula -C_{b}-H wherein C_{b} is a benzylic carbon atom. For instance, amination according to the method of the invention of 2-ethyltoluene preferably takes place at the benzylic carbon atom of the ethyl substituent, this one being most suited for the stabilization of a free radical.

In preferred embodiments of the first aspect of the invention, optionally in combination with one or more features of the various embodiments described above or below, the method of the invention is carried out at a temperature comprised from 10 °C to 50 °C. In more preferred embodiments of the first aspect of the invention, optionally in combination with one or more features of the various embodiments described above or below, the method of the invention is carried out at room temperature. Room temperature shall refer to in the context of the invention a temperature comprised from 20 °C to 25 °C.

In preferred embodiments of the first aspect of the invention, optionally in combination with one or more features of the various embodiments described above or below, the method of the invention is carried out under irradiation of visible light. In more preferred embodiments of the first aspect of the invention, optionally in combination with one or more features of the various embodiments described above or below, the method of the invention is carried out under irradiation of light, wherein the wavelength of irradiation is comprised from 400 to 700 nm. In even more preferred embodiments of the first aspect of the invention, optionally in combination with one or more features of the various embodiments described above or below, the method of the invention is carried out under irradiation of light, wherein the wavelength of irradiation is comprised from 400 to 550 nm. In even more preferred embodiments of the first aspect of the invention, optionally in combination with one or more features of the various embodiments described above or below, the method of the invention is carried out under irradiation of blue light. Suitable irradiation means, such as blue LEDs are known in the art and exhibit an emission peak at a wavelength of about 475 nm.

In preferred embodiments of the first aspect of the invention, optionally in combination with one or more features of the various embodiments described above or below, the method of the invention is carried out in an aprotic solvent that is selected from the group consisting of dichloromethane, chloroform, tetrachloroethane, dichloroethane and acetonitrile. In preferred embodiments of the first aspect of the invention, optionally in combination with one or more features of the various embodiments described above or below, the method of the invention is carried out in dichloromethane.

In other preferred embodiments of the first aspect of the invention, optionally in combination with one or more features of the various embodiments described below, the method of the invention is that wherein the molar ratio of the compound comprising the fragment of formula -Cₐ-H to the amination agent is comprised from 5:1 to 1:1. In other more preferred embodiments of the first aspect of the invention, optionally in combination with one or more features of the various embodiments described below, the method of the invention is that wherein the molar amount of the compound comprising the fragment of formula -Cₐ-H is equal to the molar amount of the amination agent. Unlike certain methods described in the state of the art, the method of the invention advantageously allows using equimolar amounts of the coupling partners, i.e. the amination agent and the compound comprising the fragment of formula -Cₐ-H.

In other preferred embodiments of the first aspect of the invention, optionally in combination with one or more features of the various embodiments described below, the method of the invention is that wherein the amination agent is selected from a compound of formula R₁SO₂NHR₂ group and a compound of formula R₁SO₂N=IR'₂ wherein R₂ is selected from the group consisting of hydrogen and a methyl group; R'₂ is phenyl, and each R₁ is independently selected from the group consisting of methyl, trifluoromethyl, *p*-tolyl and *p*-nitrophenyl.

In other preferred embodiments of the first aspect of the invention, optionally in combination with one or more features of the various embodiments described below, the method of the invention is that wherein the amination agent is a compound of formula R₁SO₂N=IR'₂ wherein R'₂ is phenyl and R₁ is p-tolyl.

In other preferred embodiments of the first aspect of the invention, optionally in combination with one or more features of the various embodiments described below, the method of the invention is that wherein the amination agent is a compound of formula R₁SO₂NH₂ wherein R₁ is as defined above. In more preferred embodiments of the first aspect of the invention, optionally in combination with one or more features of the various embodiments described below, the method of the invention is that wherein the amination agent is a compound of formula R₁SO₂NH₂ wherein R₁ is selected from the group consisting of methyl, trifluoromethyl, *p*-tolyl and *p*-nitrophenyl. In even more preferred embodiments of the first aspect of the invention, optionally in combination with one or more features of the various embodiments described below, the method of the invention is that wherein the amination agent is a compound of formula R₁SO₂NH₂ wherein R₁ is trifluoromethyl.

In other preferred embodiments of the first aspect of the invention, optionally in combination with one or more features of the various embodiments described below, the method of the invention is that wherein the amination agent is a compound of formula R₁SO₂NHR₂ wherein R₁ is as defined above and R₂ is a methyl group. In more preferred embodiments of the first aspect of the invention, optionally in combination with one or more features of the various embodiments described below, the method of the invention is that wherein the amination agent is a compound of formula R₁SO₂NHR₂ wherein R₁ is selected from the group consisting of methyl, trifluoromethyl, *p*-tolyl and *p*-nitrophenyl and R₂ is a methyl group. In even more preferred embodiments of the first aspect of the invention, optionally in combination with one or more features of the various embodiments described below, the method of the invention is that wherein the amination agent is a compound of formula R₁SO₂NH₂ wherein R₁ is selected from the group consisting of trifluoromethyl and *p*-tolyl, and wherein R₂ is a methyl group.

In other preferred embodiments of the first aspect of the invention, optionally in combination with one or more features of the various embodiments described below, the method of the invention is that wherein the amination agent is isotopically labelled. In preferred embodiments of the first aspect of the invention, optionally in combination with one or more features of the various embodiments described below, the method of the invention is that wherein the amination agent is a compound of formula R₁SO₂NH₂ as defined above wherein the nitrogen atom is an isotopic ¹⁵N atom.

In other preferred embodiments of the first aspect of the invention, optionally in combination with one or more features of the various embodiments described below, the method of the invention is that wherein, in the compound of formula (I), Ar is phenyl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy. In more preferred embodiments of the first aspect of the invention, optionally in combination with one or more features of the various embodiments described below, the method of the invention is that wherein, in the compound of formula (I), Ar is phenyl.

In other preferred embodiments of the first aspect of the invention, optionally in combination with one or more features of the various embodiments described below, the method of the invention is that wherein, in the compound of formula (I), R₃ is a radical selected from the group consisting of (C₁-C₆)haloalkyl and phenyl optionally substituted at any available position with one or more radical selected from the group consisting of halo, (C₁-C₆)alkyl, (C₁-C₆)haloalkyl, nitro, cyano, (C₁-C₆)alkyloxycarbonyl, and (C₁-C₆)alkylcarbonyloxy. In more preferred embodiments of the first aspect of the invention, optionally in combination with one or more features of the various embodiments described below, the method of the invention is that wherein, in the compound of formula (I), R₃ is a radical selected from the group consisting of trifluoromethyl and phenyl optionally substituted at any available position with a radical selected from the group consisting of methyl, fluoro, bromo and chloro. In even more preferred embodiments of the first aspect of the invention, optionally in combination with one or more features of the various embodiments described below, the method of the invention is that wherein, in the compound of formula (I), R₃ is a radical selected from the group consisting of trifluoromethyl, 3-chloro-phenyl, 4-chlorophenyl, 3-fluorophenyl, *m*-tolyl, *p-*tolyl and 4-bromophenyl.

In other preferred embodiments of the first aspect of the invention, optionally in combination with one or more features of the various embodiments described below, the method of the invention is that wherein, in the compound of formula (I), in the compound of formula (I), Ar is phenyl and R₃ is selected from the group consisting of trifluoromethyl and phenyl optionally substituted at any available position with a radical selected from the group consisting of methyl, fluoro, bromo and chloro; preferably, Ar is phenyl and R₃ is selected from the group consisting of trifluoromethyl, 3-chlorophenyl, 4-chlorophenyl, 3-fluorophenyl, *m*-tolyl, *p*-tolyl and 4-bromophenyl; more preferably, Ar is phenyl and R₃ is 4-bromophenyl.

In other preferred embodiments of the first aspect of the invention, optionally in combination with one or more features of the various embodiments described below, the method of the invention is that wherein the amount of the compound of formula (I) is twice the amount of the compound comprising the fragment of formula -Cₐ-H.

In other preferred embodiments of the first aspect of the invention, optionally in combination with one or more features of the various embodiments described below, the method of the invention is that wherein the molar ratio of compound comprising the fragment of formula -Cₐ-H to iodine is comprised of from 100:1 to 2:1. In more preferred embodiments of the first aspect of the invention, optionally in combination with one or more features of the various embodiments described below, the method of the invention is that wherein the molar ratio of compound comprising the fragment of formula -Cₐ-H to iodine is comprised of from 10:1 to 5:1. In even more preferred embodiments of the first aspect of the invention, optionally in combination with one or more features of the various embodiments described below, the method of the invention is that wherein the molar ratio of compound comprising the fragment of formula -Cₐ-H to iodine is 10:1.

The preferred embodiments described above for the method of the invention related to solvent, temperature, irradiation, compound of formulae (I) and (I), amount of iodine and molar ratios, alone or in combination one with another, are advantageous as they allow obtaining high yields for the amination product. In other preferred embodiments of the first aspect of the invention, optionally in combination with one or more features of the various embodiments described below, the method of the invention produces a compound comprising a fragment of formula -Cₐ-NSO₂R₁ from a compound comprising a fragment of formula -Cₐ-H in a yield higher than 20%, wherein said yield corresponds to the ratio of the amount of compound comprising a fragment of formula -Cₐ-NSO₂R₁ produced to the amount of compound comprising a fragment of formula -Cₐ-H engaged in the reaction. In other more preferred embodiments of the first aspect of the invention, optionally in combination with one or more features of the various embodiments described below, the method of the invention produces a compound comprising a fragment of formula -Cₐ-NSO₂R₁ from a compound comprising a fragment of formula -Cₐ-H in a yield higher than 50%.

In other preferred embodiments of the first aspect of the invention, optionally in combination with one or more features of the various embodiments described below, the method of the invention is that wherein the fragment of formula -Cₐ-H is comprised in the molecular fragments of formulae 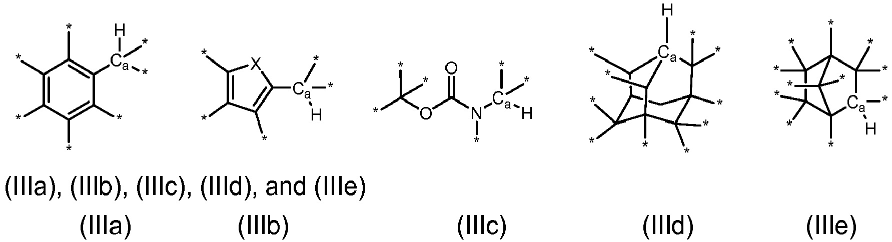 wherein the asterisks represent connections to the remainder fragments of the compound comprising the fragment of formula -Cₐ-H and X is selected from the group consisting of O, S and NR₄, being R₄ a radical selected from the group consisting of hydrogen, (C₁-C₆)alkyl, (C₁-C₆)alkylsulfonyl and (C₁-C₆)haloalkylsulfonyl.

As will be apparent to the skilled in the art, when Cₐ is a secondary or tertiary carbon atom, the method of the invention may produce a chiral aminated product bearing a chiral center at Cₐ. This is particularly the case when Cₐ is substituted in the compound comprising the fragment of formula-Cₐ-H with at least two groups other than hydrogen that are different one with another. Thus, in further embodiments of the first aspect of the invention, optionally in combination with one or more features of the various embodiments described below, the method of the invention further comprises the step of submitting the compound comprising the fragment of formula -Cₐ-NHSO₂R₁ to a kinetic resolution method producing an enantiomerically enriched compound comprising the fragment of formula -Cₐ-NHSO₂R₁. Such methods are known in the art, and have been reported for instance by Chu and co-workers.

In further embodiments of the first aspect of the invention, optionally in combination with one or more features of the various embodiments described below, the method of the invention further comprises the step of converting the compound comprising the fragment of formula -Cₐ-NSO₂R₁ in a compound comprising a fragment of formula -Cₐ-NH₂. The skilled in the art person shall easily find suitable conditions in order to produce the free amine from the sulfonamide compound using common general knowledge. Different sets of reaction conditions are known in the art to carry out this transformation, such as basic hydrolysis, hydrogenolysis and reduction with hydride species.

The method of the first aspect of the invention is useful for the introduction of an amino group at a determined position of a reaction substrate. Amines, and more particularly benzylic amines, are frequent molecular fragments in biologically active natural products and active pharmaceutical ingredients. The method of the invention is therefore useful as a step in the preparation of a biologically active ingredient. According to the second aspect of the invention, the invention relates to a method for the preparation of a biologically active compound or a salt thereof comprising the step of carrying out the photochemical method according to the first aspect of the invention as described above.

In a preferred embodiment of the second aspect of the invention, the invention relates to a method for the preparation of a biologically active compound that is selected from the group consisting of
phenytoin, clotrimazole, etomidate, indinavir, chlorthalidone, piperacillin, cetirizine, buclizine, mirtazapine, ampicillin, metocurine iodide, cefpiramide, loracarbef, vancomycin, mephenytoin, hydroxyzine, cephalexin, cinnarizine, mazindol, aprepitant, cephaloglycin, apomorphine, meclizine, hetacillin, epinastine, ethotoin, clopidogrel, tadalafil, cefaclor, levamisole, repaglinide, mezlocillin, ezetimibe, rivastigmine, letrozole, cinacalcet, praziquantel, amoxicillin, azlocillin, sertraline, doxacurium, cefadroxil, cefprozil, cyclizine, tubocurarine, ketamine, mivacurium, paclitaxel, docetaxel, fosphenytoin, metocurine, rasagiline, almitrine, ketazolam, bacampicillin, pivampicillin, bifonazole, tetrabenazine, trabactedin, oritavancin, mianserin, prasugrel, fosaprepitant, cabazitaxel, noscapine, pasireotide, chlorcyclizine, maraviroc, trimebutine, apremilast, atracurium besylate, rolapitant, ombitasvir, levocetirizine, solifenacin, quinupristin, hexafluronium, esketamine, tritoqualine, manidipine, velpatasvir ,telavancin, alazoparib, reserpine, tadalafil, deserpidine, rescinnamine, yohimbine, pirlindole, aptazapine, vincamine, valbenazine, elagolix, letermovir, sultamicillin, deutetrabenazine, cisatracurium, memantine, MK-801, strictamine, vincorine, aspidophylline A, scholarisine A, rhazimal, geissoschizine, cathafoline, pseudoakuammigine, corymine, arbophylline, nareline, picraline, echitamine, vincarinine, alstiphyllanine, alstolactine, alstoscholarine, lanciferine, alschomine, pleiocarpamine, picrinine, and akuamminiline.

In a preferred embodiment of the second aspect of the invention, the invention relates to a method for the preparation of a biologically active compound wherein the biologically active compound is selected from the group consisting of strictamine, vincorine, aspidophylline A, scholarisine A, rhazimal, geissoschizine, cathafoline, pseudoakuammigine, corymine, arbophylline, nareline, picraline, echitamine, vincarinine, alstiphyllanine, lanciferine, alschomine, pleiocarpamine, picrinine, akuamminiline, maraviroc, apremilast, memantine, rasagiline, sertraline, MK-801, repaglinide, cinacalcet, prasugrel, flunarizine, solifenacin, aryl glycine, and hexafluronium. Reserpine, tadalafil, deserpidine, rescinnamine, yohimbine, pirlindole, aptazapine, vincamine, strictamine, vincorine, aspidophylline A, scholarisine A, rhazimal, alstolactine, alstoscholarine, geissoschizine, cathafoline, pseudoakuammigine, corymine, arbophylline, nareline, picraline, echitamine, vincarinine, alstiphyllanine, lanciferine, alschomine, pleiocarpamine, picrinine, and akuamminiline may be produced by a process comprising the step of carrying out the method of the invention wherein the fragment of formula -Cₐ-H is a fragment of formula (IIIb) as defined above wherein X is NR₄. Phenytoin, clotrimazole, etomidate, indinavir, chlorthalidone, piperacillin, cetirizine, buclizine, mirtazapine, ampicillin, metocurine iodide, cefpiramide, loracarbef, vancomycin, mephenytoin, hydroxyzine, cephalexin, cinnarizine, mazindol, aprepitant, cephaloglycin, apomorphine, meclizine, hetacillin, epinastine, ethotoin, clopidogrel, tadalafil, cefaclor, levamisole, repaglinide, mezlocillin, ezetimibe, rivastigmine, letrozole, cinacalcet, praziquantel, amoxicillin, azlocillin, sertraline, doxacurium, cefadroxil, cefprozil, cyclizine, tubocurarine, ketamine, mivacurium, paclitaxel, docetaxel, fosphenytoin, metocurine, rasagiline, almitrine, ketazolam, bacampicillin, pivampicillin, bifonazole, tetrabenazine, trabactedin, oritavancin, mianserin, prasugrel, fosaprepitant, cabazitaxel, noscapine, pasireotide, chlorcyclizine, maraviroc, trimebutine, apremilast, atracurium besylate, rolapitant, ombitasvir, levocetirizine, solifenacin, quinupristin, hexafluronium, esketamine, tritoqualine, manidipine, velpatasvir ,telavancin, alazoparib, valbenazine, elagolix, letermovir, sultamicillin, deutetrabenazine, cisatracurium, and MK-801 may be produced by a process comprising the step of carrying out the method of the invention wherein the fragment of formula -Cₐ-H is a fragment of formula (IIIa) as defined above.

Memantine may be produced by a process comprising the step of carrying out the method of the invention wherein the fragment of formula -Cₐ-H is a fragment of formula (IIId) as defined above.

In a preferred embodiment of the second aspect of the invention, the invention relates to a method for the preparation of a biologically active compound wherein the biologically active compound is selected from the group consisting of memantine, rasagiline, sertraline and MK-801.

Memantine may be prepared according to the method comprising the steps of:
(i) submitting 1,3-dimethyladamantane to the method of the first aspect of the invention, producing a sulfonylated 3,5-dimethyladamantan-1-amine compound; and
(ii) submitting the product of step (i) to a desulfonylation method, preferably by treatment with a base such as cesium carbonate, producing 3,5-dimethyladamantan-1-amine.

The synthetic scheme below describes said process: 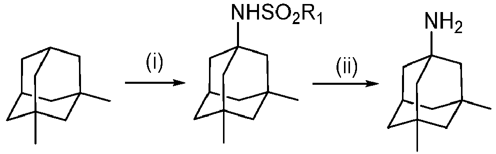 Optionally, the product resulting of step (ii) may be further treated with an acid to produce a salt. When the acid is hydrochloric acid, memantine hydrochloride is produced.

Rasagiline may be prepared according to the method comprising the steps of:
(i) submitting 2,3-dihydroindene to the method of the first aspect of the invention, producing a sulfonylated 2,3-dihydro-1H-inden-1-amine compound;
(ii) contacting the product of step (i) with a compound formula HCCCH₂Y, being Y a leaving group, producing a sulfonylated N-(prop-2-yn-1-yl)-2,3-dihydro-1H-inden-1-amine compound; and
(iii) submitting the product of step (ii) to a desulfonylation method, preferably by treatment with a hydride source, such as lithium aluminium hydride.

The synthetic scheme below describes said process:

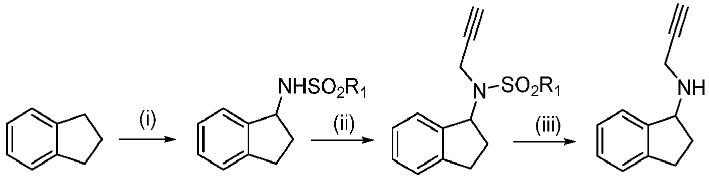

Optionally, the product resulting of step (i), (ii) or (iii) may be further submitted to a kinetic resolution method to produce an enantiomerically enriched compound.

Sertraline may be prepared according to the method comprising the steps of:
(i) submitting 3,4-dihydronaphthalen-1(2H)-one to the method of the first aspect of the invention, producing a sulfonylated 4-amino-3,4-dihydronaphthalen-1(2H)-one compound;
(ii) contacting the product of step (i) with a methylating agent, such as methyl iodide, producing a sulfonylated 4-(methylamino)-3,4-dihydronaphthalen-1(2H)-one compound;
(iii) submitting the product of step (ii) to reactions conditions sufficient to convert the ketone group of the product of step (ii) in an enol group, wherein the hydroxyl group is replaced by a leaving group Z; such as a halo, a tosylate or a triflate;
(iv) contacting the product of step (iii) with (3,4-dichlorophenyl)boronic acid under reactions of conditions sufficient for a cross-coupling to take place, producing a sulfonylated 4-(3,4-dichlorophenyl)-N-methyl-1,2-dihydronaphthalen-1-amine compound;
(v) submitting the product of step (iv) to a hydrogenation step, producing a sulfonylated 4-(3,4-dichlorophenyl)-N-methyl-1,2,3,4-tetrahydronaphthalen-1-amine compound; and
(vi) submitting the product of step (v) to a desulfonylation method, preferably by treatment with a hydride source, such as lithium aluminium hydride.

The synthetic scheme below describes said process:

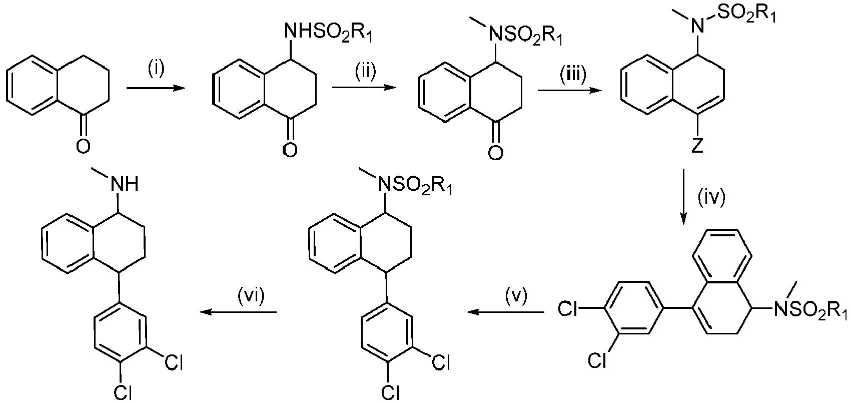

Optionally, the product resulting of step (i), (ii), (iii), (iv), (v) or (vi) may be further submitted to a kinetic resolution method to produce an enantiomerically enriched compound.

MK-801 may be prepared according to the method comprising the steps of:
(i) submitting 5-methyl-10,11-dihydro-5H-dibenzo[a,d][7]annulene to the method of the first aspect of the invention, producing a sulfonylated 5-methyl-10,11-dihydro-5H-5,10-epiminodibenzo[a,d][7]annulene compound; and
(ii) submitting the product of step (i) to a desulfonylation method, preferably by treatment with a hydride source; producing 5-methyl-10,11-dihydro-5H-5,10-epiminodibenzo[a,d][7]annulene.

The synthetic scheme below describes said process:

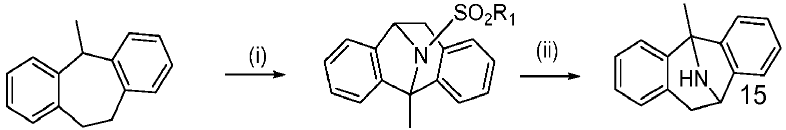

Certain indole-based alkaloids, such as strictamine, alstolactine A, scholarisine K and altoscholarine may be prepared from a common synthetic intermediate of formula (III) wherein R₅ is selected from hydrogen and a nitrogen protecting group (e.g. carbamate, sulfonamide, benzyl, amide).

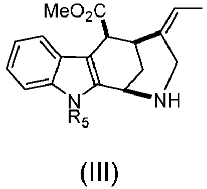

The compound of formula (III) may be prepared according the synthetic scheme described below, wherein the step (i) is the method of the first aspect 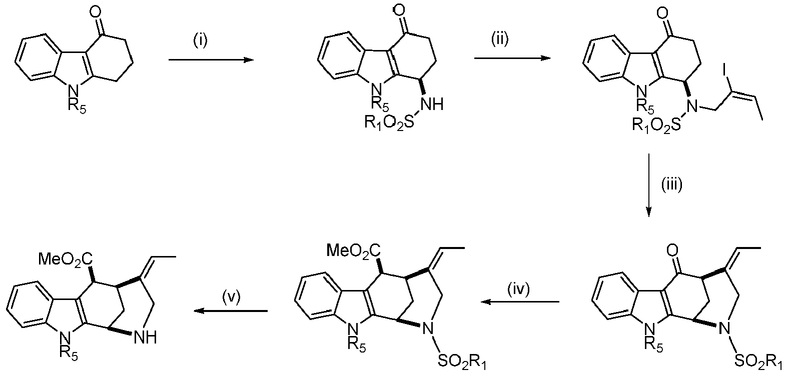 of the invention.

Suitable methods are known in the art to carry out steps (ii), (iii), (iv) and (v) and shall be apparent to the skilled in the art using common general knowledge. Step (ii) may for instance be carried out by contacting the product of step (i) with a compound of formula Z-CH₂-CH(I)=CH(CH₃) being Z a leaving group. Step (iii) may be carried out using suitable reaction conditions for allylic substitution reactions, for instance through the use of catalytic amounts of palladium. Step (iv) may be carried out using a sequence of a Wittig reaction producing a carbon-carbon double bond from the carbonyl group followed by an oxidation step (e.g. with ozone) leading to the carboxylic acid that may subsequently undergo esterification. Step (v) may be carried out using suitable means for desulfonylation of the nitrogen atom, such as treatment with a base (e.g. cesium or potassium carbonate) or treatment with a hydride source. The compound of formula (III) may further be used as synthetic precursor of naturally occurring indole-based alkaloids and analogues thereof.

In another preferred embodiment of the second aspect of the invention, the invention relates to a process for the preparation of pyrrolidine compounds comprising the steps of:
(i) submitting a compound comprising a fragment of formula (III) to the method of the first aspect of the invention 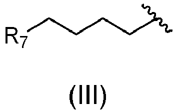 wherein R₇ is an optionally substituted aromatic or heteroaromatic group; preferably R₇ is a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy; more preferably R₇ is a phenyl group, and wherein the wavy line indicates the position where the fragment of formula (III) is connected to the remainder of the molecule;
(ii) submitting the product of step (i) to conditions of reaction sufficient to produce a compound comprising a fragment of formula (IV).

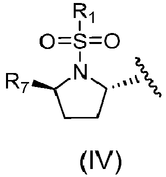

Sufficient conditions of reaction for step (ii) are known in the art and have been described for instance in Martinez and co-workers (incorporated herein by reference). Such conditions comprise contacting the product of step (i) in the presence of a catalytically effective amount of iodine with a hypervalent iodine compound of formula Phl(mCBA)₂, wherein mCBA means 3-chlorobenzoate.

Throughout the description and claims the word "comprises" and variations of the word, are not intended to exclude other technical features, additives, components or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Preparative Examples

### A) Synthesis of Trifluoro-N-methylmethanesulfonamide

A solution of trifluoromethanesulfonyl chloride (842 mg, 5.00 mmol, 1.25 equiv) in THF (2.5 mL), was added to as solution of methylamine hydrochloride (270 mg, 4.00 mmol,1.0 equiv) in THF (2 mL) at 0 °C. The reaction mixture was stirred for 5 min, and an aqueous solution of NaOH (10M, 0.4 mL) was added. The reaction was stirred for 2 h at 0 °C. The reaction mixture was warmed to r.t. and 1N HCl aqueous solution was added. The organic phase was extracted with EtOAc. The combined organic phases were dried over Na2SO4. After filtration, the solvent was evaporated under reduced pressure, to yield the product as brownish oil in 72% yield.

### Example 1: Amination of adamantane

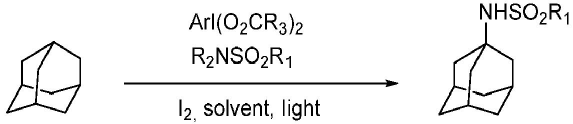

A Schlenk tube equipped with a stirrer bar is charged with adamantane (0.3 mmol), the compound of formula Arl(O₂CR₃)₂ (1.0-2.0 equiv, as indicated in Table 1), I₂ (0-0.1 equivalent as indicated in Table 1) and the compound of formula R₂NSO₂R₁ (1.0 equiv), evacuated, and backfilled with argon, before 1.5 mL of solvent (as indicated in Table 1) are added. The solution is stirred at 25 °C for 12h under visible light as indicated in Table 1. Solvent was then evaporated and crude mixture was analyzed by 1H NMR analysis to determine the yield of the reaction, using 1,3,5-trimethoxybenzene as internal standard.

Table 1 summarizes the results obtained in different experiments varying the reagents and conditions of reaction.

**Table 1**

| Entry | R₂NSO₂R₁ (1 equivalent) | Arl(O₂CR₃)₂ (amount) | Iodine amount | Solvent | Light source (nm) | Yield (%) |
|---|---|---|---|---|---|---|
| 1 | HNTs₂ | Phl(CBA)₂ (1.1) | 0.1 | CH₂Cl₂ | 400-700 | 0 |
| 2 | H₂NTs | Phl(CBA)₂ (2.0) | 0.1 | (CH₂Cl)₂ | 400-700 | 38 |
| 3 | H₂NTs | Phl(CBA)₂ (2.0) | 0.2 | CH₂Cl₂ | 400-700 | 50 |
| 4 | H₂NTs | Phl(CBA)₂ (2.0) | 0 | CH₂Cl₂ | 400-700 | 0 |
| 5 | H₂NTs | Phl(CBA)₂ (2.0) | 0.1 | CHCl₃ | 400-700 | 30 |
| 6 | H₂NTs | Phl(CBA)₂ (2.0) | 0.1 | CH₃CN | 400-700 | 15 |
| 7 | H₂NTs | Phl(CBA)₂ (2.0) | 0.1 | EtOAc | 400-700 | 0 |
| 8 | H₂NTs | Phl(CBA)₂ (2.0) | 0.1 | CH₂Cl₂ | 400-700 | 34 |
| 9 | H₂NTs | Phl(CBA)₂ (2.0) | 0.1 | CH₂Cl₂ | 400 (purple LED) | 30 |
| 10 | H₂NTs | Phl(CBA)₂ (2.0) | 0.1 | CH₂Cl₂ | 475(blue LED) | 35 |
| 11 | H₂NTs | Phl(CBA)₂ (2.0) | 0.1 | CH₂Cl₂ | 510 (Green LED) | 20 |
| 12 | H₂NTs | Phl(CBA)₂ (2.0) | 0.1 | CH₂Cl₂ | Dark lab (none) | 10 |
| 13 | Phl=NTs | Phl(CO₂CF₃)₂ (2.0) | 0.1 | CH₂Cl₂ | 475 | 60 |
| 14 | H₂NNs | Phl(CO₂CF3)₂ (2.0) | 0.1 | CH₂Cl₂ | 475 | 30 |
| 15 | H₂NMs | Phl(CO₂CF3)₂ (2.0) | 0.1 | CH₂Cl₂ | 475 | 65 |
| 16 | MeONH₂ | Phl(CO₂CF3)₂ (2.0) | 0.1 | CH₂Cl₂ | 475 | 0 |
| 17 | tBuNH₂ | Phl(CO₂CF3)₂ (2.0) | 0.1 | CH₂Cl₂ | 475 | 0 |
| 18 | PhthNH₂ | Phl(CO₂CF3)₂ (2.0) | 0.1 | CH₂Cl₂ | 475 | 0 |
| 19 | H₂NTf | Phl(CO₂CF3)₂ (2.0) | 0.1 | CH₂Cl₂ | 475 | 70 |
| 20 | H₂NTf | Phl(CBA)₂ (2.0) | 0.1 | CH₂Cl₂ | 475 | 99 |

| | | | | | | |
|---|---|---|---|---|---|---|
| Amounts are expressed in equivalents of adamantane. CBA=3-chlorobenzoate; Ts = p-toluenesulfonyl, Ms=methylsulfonyl, Tf=trilfuoromethylsulfonyl, Ns=p-nitrophenylsulfonyl | | | | | | |

Entries 1, 4, 7, 16, 17 and 18 correspond to embodiments falling out of the scope of the invention and are comparative examples that indicate that good yields are obtained when conditions of reaction falling within the scope of the invention are implemented.

### Example 2: Amination of ethylbenzene

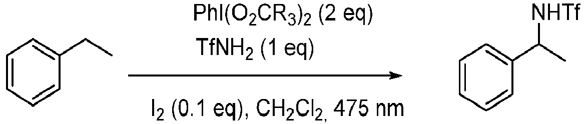

A Schlenk tube equipped with a stirrer bar is charged with ethylbenzene (0.3 mmol), the compound of formula Phl(O₂CR₃)₂ (2.0 equiv), I₂ (0.1 equivalent) and trifluoromethylsulfonamide (1.0 equiv), evacuated, and backfilled with argon, before 1.5 mL of dichloromethane are added. The solution is stirred at 25 °C for 12h under blue light irradiation (475 nm, blue LED). Solvent was then evaporated and crude mixture was analyzed by ¹H NMR analysis to determine the yield of the reaction, using 1,3,5-trimethoxybenzene as internal standard.

Table 2 summarizes the results obtained in different experiments varying the temperature and the compound of formula Arl(O₂CR₃)₂.

**Table 2**

| Entry | R₃CO₂- | Temperature (°C) | Yield (%) |
|---|---|---|---|
| 1 | 3-chlorobenzoate | 25 | 52 |
| 2 | 4-chlorobenzoate | 25 | 55 |
| 3 | 3-fluorobenzoate | 25 | 45 |
| 4 | 3-methylbenzoate | 25 | 24 |
| 5 | 4-trifluoromethylbenzoate | 25 | 55 |
| 6 | Benzoate | 25 | 72 |
| 7 | 4-methylbenzoate | 25 | 38 |
| 8 | 4-bromobenzoate | 25 | 80 |
| 9 | 4-bromobenzoate | 45 | 92 |

The combinations of Table 1 and Table 3 teach the best mode of carrying out the invention.

### Example 3: Amination of various substrates

General procedure 1: The corresponding starting material (0.2 mmol, 1.0 equiv), trifluoromethanesulfonamide (30 mg, 0.2 mmol, 1.0 equiv), I₂ (5 mg, 0.02 mmol, 10 mol%) and Phl(4-bromobenzoate)₂ (241 mg, 0.40 mmol, 2.0 equiv) were charged into a dried Schlenk tube (evacuated and backfilled with argon), equipped with a magnetic stir bar. 2 mL of absolute dichloromethane were added and the reaction mixture was irradiated under blue LEDs at room temperature. After 14h, dichloromethane was added and the mixture was washed with a saturated aqueous solution of Na₂S₂O₃ and then with a saturated aqueous solution of NaHCO₃. The combined organic phases were dried over Na₂SO₄ and the solvent was removed under reduced pressure. The crude material was purified by column chromatography (SiO₂, *n*-hexane/EtOAc) to give the pure product.

Table 3 summarizes the results obtained for the preparation of various aminated products following general procedure 1. Starting materials were either purchased from commercial sources or prepared from commercial materials using literature procedures.

**Table 3**

| Starting material | Product | Yield (%) | Starting material | Product | Yield (%) |
|---|---|---|---|---|---|
| 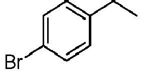 | 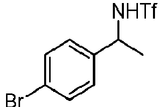 | 85 | 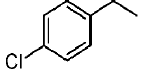 | 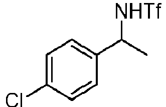 | 82 |
| 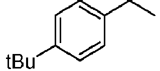 | 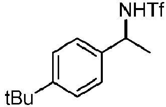 | 87 | 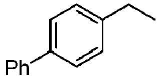 | 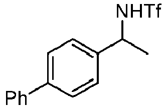 | 67 |
| 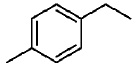 | 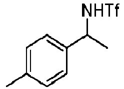 | 85 | 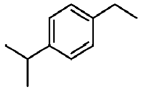 | 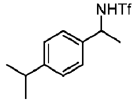 | 60 |
| 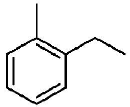 | 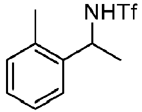 | 70 | 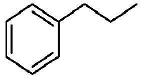 | 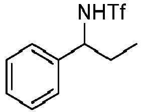 | 68 |
| 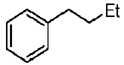 | 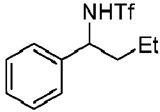 | 63 | 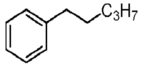 | 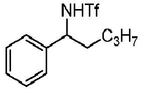 | 57 |
| 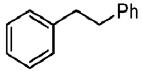 | 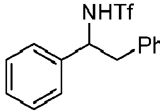 | 73 | 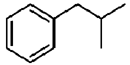 | 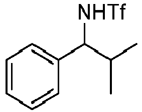 | 67 |
| 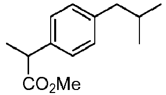 | 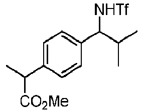 | 72 | 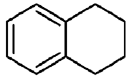 | 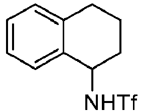 | 65 |
| 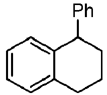 | 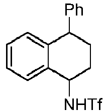 | 63 | 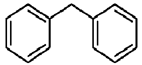 | 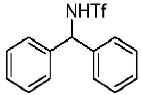 | 71 |
| 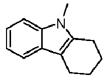 | 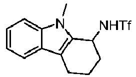 | 68 | 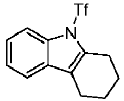 | 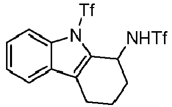 | 62 |
| 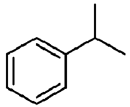 | 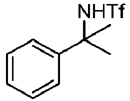 | 52 | 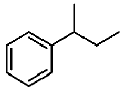 | 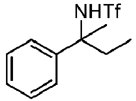 | 50 |
| 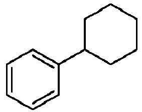 | 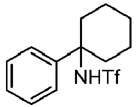 | 62 | 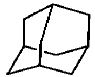 | 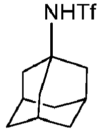 | 95 |
| 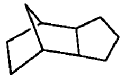 | 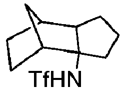 | 65 | 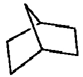 | 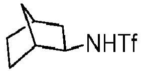 | 70 |
| 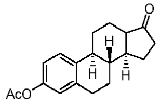 | 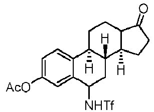 | 72 | 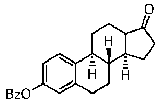 | 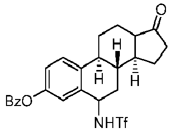 | 65 |
| 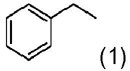 | 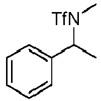 | 45 | 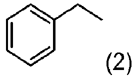 | 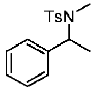 | 32 |
| 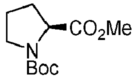 | 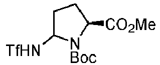 | 68 | 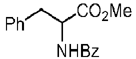 | 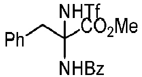 | 59 |
| 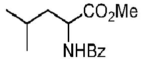 | 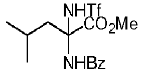 | 65 | 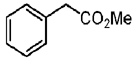 | 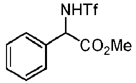 | 40 |
| 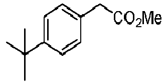 | 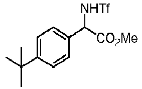 | 35 | 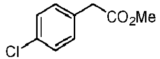 | 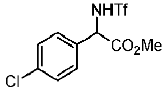 | 42 |
| 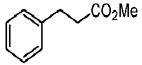 | 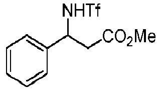 | 68 | 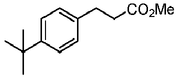 | 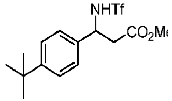 | 72 |
| 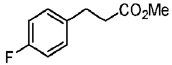 | 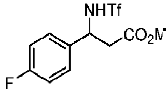 | 73 | 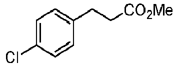 | 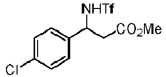 | 65 |

| | | | | | |
|---|---|---|---|---|---|
| (1) N-methyl-trifluorosulfonamide was used instead of trifluorosulfonamide. (2) N-methyl-4-methylphenylsulfonamide was used instead of trifluorosulfonamide. | | | | | |

Table 3 indicates that the method of the invention is applicable to a broad range of substrates.

The free amine may further be obtained following suitable desulfonylation methods as known in the art. When the obtained sulfonamide is a tertiary sulfonamide, suitable procedures for desulfonylation are described in general procedures 2 and 3. When the obtained sulfonamide is a secondary sulfonamide, suitable procedures for desulfonylation are described in general procedure 4.

General Procedure 2: A flame-dried Schlenk tube (evacuated and backfilled with argon), equipped with a magnetic stir bar was charged with the corresponding aminated product (1.0 equiv) dissolved in dry toluene (1 mL/ 0.1 mmol) and cooled to 0 °C. A solution of Red-AI (60% in toluene, 10 equiv) was added drop-wise. The mixture was then heated at 50 °C for 14h. A solution of NH₄Cl (5% aq) was added and the organic phase was separated. The organic phase was dried over Na₂SO₄ and the solvent was removed under reduced pressure. The crude material was purified by column chromatography (SiO₂, *n*-hexane/EtOAc) to give the pure product.

General Procedure 3: A flame-dried Schlenk equipped with a magnetic stir bar and a reflux condenser was charged with LiAlH₄ (5.0 equiv), anhydrous toluene was added carefully and the mixture was cooled to 0 °C. The corresponding aminated product (1.0 equiv) was dissolved in dry toluene (5 mL/mmol) and was added dropwise to the LiAlH₄/toluene suspension under an argon atmosphere. The mixture was then heated to reflux for 5 h and cooled to 0 °C. An aqueous solution of NaOH (1 M) was added. After filtration over Na₂SO₄ and evaporation of the solvent under reduced pressure, the amine was obtained in quantitative yield.

General procedure 4: The corresponding aminated substrate (1.0 equiv), K₂CO₃ (1.2 equiv), 4-nitrobenzylbromide (1.2 equiv) were added in a round bottom flask equipped with a magnetic stir bar. Acetone (1 mL/ 0.1 mmol) was added and the suspension was stirred at room temperature overnight. After evaporation of the solvent H₂O was added and the mixture was extracted with EtOAc. The organic phase was dried over Na₂SO₄ and the solvent was removed under reduced pressure. The crude material was dissolved in THF (1 mL/0.1 mmol) and Cs₂CO₃ (2.0 equiv) was added. The suspension was stirred at 50 °C for 14h. The reaction mixture was then acidified with aqueous HCl (1 M), and the THF was evaporated under reduced pressure. The aqueous phase was washed with Et₂O. The aqueous phase was evaporated under reduced pressure to yield the pure product.

### Example 4: Synthesis of sertraline

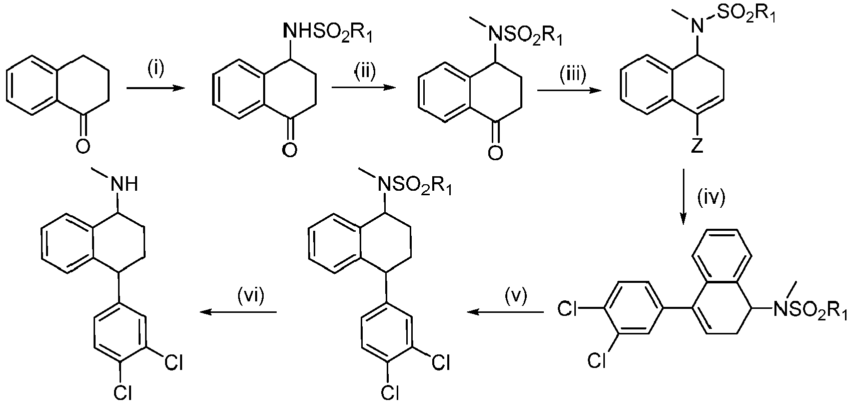

(i) 1,1,1-Trifluoro-N-(4-oxo-1,2,3,4-tetrahydronaphthalen-1-yl)methanesulfonamide was produced in 55% yield from α-tetralone following general procedure 1.
(ii)-(vi) 4-(3,4-Dichlorophenyl)-N-methyl-1,2,3,4-tetrahydronaphthalen-1-amine was produced in 52% overall yield after the following sequence: A) methylation of the sulfonamide with methyl iodide, B) formation of the enoltriflate, C) Suzuki coupling with 3,4-dichlorophenylboronic acid, D) Catalytic Hydrogenation over a palladium catalyst, E) Deprotection for tertiary sulfonamides following general procedure 3.

### Example 5: Synthesis of memantine

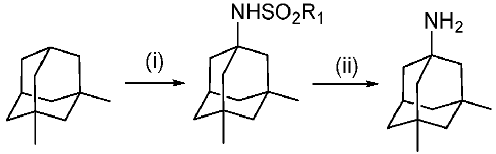

(i) N-((1,3,5,7)-3,5-Dimethyladamantan-1-yl)-1,1,1-trifluoromethanesulfonamide was produced in 92% yield from 1,3-dimethyladamantane following general procedure 1.
(ii) 1,3-Dimethyladamantanamine hydrochloride was produced in 92% yield from the product of step (i) following general procedure 4 followed by a treatment with hydrochloric acid.

### Example 6: Synthesis of rasagiline

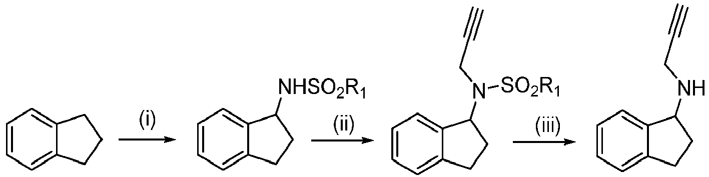

(i) N-(2,3-dihydro-1H-inden-1-yl)-1,1,1-trifluoromethanesulfonamide was produced in 78% yield from indane following general procedure 1.
(ii) N-(2,3-Dihydro-1H-inden-1-yl)-1,1,1-trifluoro-N-(prop-2-yn-1-yl)methanesulfonamide was prepared following procedures known in the art in 99% yield by treatment with 3-bromoprop-1-yne.
(iii) N-(Prop-2-yn-1-yl)-2,3-dihydro-1H-inden-1-amine was produced in 78% yield from the product of step (i) following general procedure 3.

### Example 7: Synthesis of pyrrolidine compounds

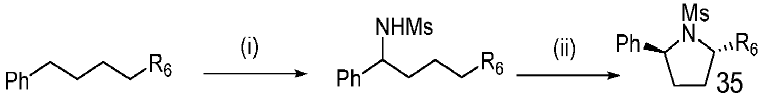

Step (i) corresponds to general procedure 1, replacing trifluoromethylsulfonamide by methylsulfonamide.
Step (ii) corresponds to general procedure 5 as described below and in Martinez and co-workers.

General procedure 5: The corresponding starting material (0.2 mmol, 1.0 equiv), I₂ (5 mg, 0.02 mmol, 10 mol%) and Phl(mCBA)₂ (113 mg, 0.22 mmol, 1.1 equiv - mCBA = m-chlorobenzoate) were charged into a dried Schlenk tube (evacuated and backfilled with argon), equipped with a magnetic stir bar. 2 mL of absolute dichloroethane were added and the reaction mixture was irradiated under white LEDs at room temperature. After 14 h, dichloromethane was added and the mixture was washed with an aqueous solution of Na₂S₂O₃ and then with a saturated aqueous solution of NaHCO₃. The combined organic phases were dried over Na₂SO₄ and the solvent was removed under reduced pressure. The crude material was purified by column chromatography (SiO₂, n-hexane/EtOAc) to give the pure product.
Table 4 summarizes the results obtained with several R₆ groups.

**Table 4**

| R₆ | Yield of step (i) (%) | Yield of step (ii) (%) |
|---|---|---|
| H | 35 | 56 |
| Me | 30 | 59 |
| Isopropyl | 30 | 60 |
| phenyl | 29 | 87 |

When R₆ is a phenyl group, the desired pyrrolidine compound may be obtained directly from the starting material following general procedure 1 wherein twice the amount of iodine and the compound of formula (I) are used.

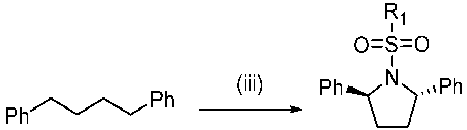

Table 5 summarizes such experiments with different R₁ groups in the amination agent.

**Table 5**

| R₁ | Me | p-tolyl | CF₃ |
|---|---|---|---|
| Yield of pyrrolidine (%) | 68 | 52 | 85 |

### CITATION LIST

1. Bergès, J.; Garcia B.; Muñiz, K. Angew. Chem. Int. Ed. 2018, 57, 15891 - 15895.
2. Duhamel, T.; Stein, C.; Martínez, C.; Reiher, M.; Muñiz, K. ACS Catal. 2018, 8, 3918-3925.
3. Souto, J.A.; Zian, D.; Muñiz, K. J. Am. Chem. Soc. 2012, 134, 7242-7245.
4. Lamar, A.A.; Nicholas, K.M. J. Org. Chem. 2010, 75, 7644-7650.
5. Fan, R.; Li, W.; Pu, D.; Zhang, L. Org. Lett. 2009, 11(6), 1425-1428.
6. Chu, L.; Xiao, K.-J.; Yu, J.-Q. Science 2014, 346, 451-455.
7. Martinez, C.; Muñiz, K. Angew. Chem., Int. Ed. 2015, 54, 8287.

## Claims

1. A photochemical method for the intermolecular coupling of an amination agent with a compound comprising a fragment of formula -Cₐ-H, said method converting the fragment of formula -Cₐ-H in a fragment of formula -Cₐ-NSO₂R₁, and comprising the step of contacting under light irradiation and in an aprotic solvent an amination agent with a compound comprising a fragment of formula -Cₐ-H in the presence of a compound of formula (I)
Arl(O₂CR₃)₂ (I)
and a catalytically effective amount of iodine, wherein:
the amination agent is selected from the compounds of formula R₁SO₂NHR₂ and the compounds of formula R₁SO₂N=IR₂' wherein:
R₁ is a radical selected from the group consisting of (C₁-C₆)alkyl, (C₁-C₆)haloalkyl, and (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy;
R₂ is selected from hydrogen and a (C₁-C₆)alkyl group;
R₂' a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy;
Cₐ is a saturated carbon atom;
Ar is a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxy, (C₁-C₆)alkyloxycarbonyl, and (C₁-C₆)alkylcarbonyloxy;
R₃ is a radical selected from the group consisting of (C₁-C₆)haloalkyl and a (C₆-C₂₀)aryl optionally substituted at any available position with one or more radical selected from the group consisting of halo, (C₁-C₆)alkyl, (C₁-C₆)haloalkyl, nitro, cyano, (C₁-C₆)alkyloxycarbonyl, and (C₁-C₆)alkylcarbonyloxy;
and the amount of the compound of formula (I) is at least twice the amount of the compound comprising the fragment of formula -Cₐ-H.

2. The photochemical method according to claim 1 wherein Cₐ is essentially the carbon atom of the compound comprising a fragment of formula -Cₐ-H that is most suited for the stabilization of a free radical.

3. The photochemical method according to any one of claims 1 and 2 wherein Cₐ is a benzylic carbon atom.

4. The photochemical method according to any one of claims 1 and 2 wherein, in the compound comprising a fragment of formula -Cₐ-H, Cₐ is a carbon atom adjacent to the nitrogen atom of a functional group selected from the group consisting of amido, carbamate, sulfonamide and phosphoramide.

5. The photochemical method according to any one of claims 1 to 4 that is carried out at room temperature.

6. The photochemical method according to any one of claims 1 to 5 that is carried out under irradiation of blue light.

7. The photochemical method according to any one of claims 1 to 6 wherein the aprotic solvent is selected from the group consisting of dichloromethane, chloroform, tetrachloroethane, dichloroethane and acetonitrile.

8. The photochemical method according to any one of claims 1 to 7 wherein the molar ratio of the compound comprising the fragment of formula -Cₐ-H to the amination agent is comprised from 5:1 to 1:1; preferably, the molar amount of the compound comprising the fragment of formula -Cₐ-H is equal to the molar amount of the amination agent.

9. The photochemical method according to any one of claims 1 to 8 wherein the amination agent is selected from a compound of formula R₁SO₂NHR₂ group and a compound of formula R₁SO₂=IR'₂ wherein R₂ is selected from the group consisting of hydrogen and a methyl group; R'₂ is phenyl, and each R₁ is independently selected from the group consisting of methyl, trifluoromethyl, *p*-tolyl and *p*-nitrophenyl; preferably, the amination agent is a compound of formula R₁SO₂NH₂ wherein R₁ is selected from the group consisting of methyl, trifluoromethyl, *p*-tolyl and *p*-nitrophenyl.; and, more preferably, the amination agent is a compound of formula R₁SO₂NH₂ wherein R₁ is trifluoromethyl.

10. The photochemical method according to any one of claims 1 to 9 wherein, in the compound of formula (I), Ar is phenyl and R₃ is selected from the group consisting of trifluoromethyl and phenyl optionally substituted at any available position with a radical selected from the group consisting of methyl, fluoro, bromo and chloro; preferably, Ar is phenyl and R₃ is selected from the group consisting of trifluoromethyl, 3-chloro-phenyl, 4-chlorophenyl, 3-fluorophenyl, *m*-tolyl, *p*-tolyl and 4-bromophenyl; more preferably, Ar is phenyl and R₃ is 4-bromophenyl.

11. The photochemical method according to any one of claims 1 to 10 wherein the amount of the compound of formula (I) is twice the amount of the compound comprising the fragment of formula -Cₐ-H.

12. The photochemical method according to any one of claims 1 to 11 wherein the molar ratio of compound comprising the fragment of formula -Cₐ-H to iodine is comprised of from 100:1 to 2:1; preferably, the molar ratio of compound comprising the fragment of formula -Cₐ-H to iodine is comprised of from 10:1 to 5:1; more preferably, the molar ratio of compound comprising the fragment of formula -Cₐ-H to iodine is 10:1.

13. The photochemical method according to any one of claims 1 to 12 further comprising the step of submitting the compound comprising the fragment of formula -Cₐ-NSO₂R₁ to a kinetic resolution method producing an enantiomerically enriched compound comprising the fragment of formula -Cₐ-NSO₂R₁.

14. The photochemical method according to any one of claims 1 to 13 further comprising the step of converting the compound comprising the fragment of formula -Cₐ-NSO₂R₁ in a compound comprising a fragment of formula -Cₐ-NH₂.

15. A method for the preparation of a biologically active compound or a salt thereof comprising the step of carrying out the photochemical method according to any one of claims 1 to 14, wherein the biologically active compound is preferably selected from the group consisting of strictamine, vincorine, aspidophylline A, scholarisine A, rhazimal, geissoschizine, cathafoline, pseudoakuammigine, corymine, arbophylline, nareline, picraline, echitamine, vincarinine, alstiphyllanine, lanciferine, alschomine, pleiocarpamine, picrinine, akuamminiline, maraviroc, apremilast, memantine, rasagiline, sertraline, MK-801, repaglinide, cinacalcet, prasugrel, flunarizine, solifenacin, aryl glycine, and hexafluronium.
